(19) Europäisches Patentamt
European Patent Office
Office européen des brevets

(11) Publication number: 0 453 119 A1

# (12) EUROPEAN PATENT APPLICATION

(21) Application number: 91302843.7

(22) Date of filing: 02.04.91

(51) Int. Cl.$^5$: C07K 13/00, C12N 15/12, C12N 15/85, A01K 67/00, A61K 31/05, C12N 15/00

(30) Priority: 03.04.90 US 503650

(43) Date of publication of application: 23.10.91 Bulletin 91/43

(84) Designated Contracting States: CH DE FR GB IT LI NL

(71) Applicant: MERCK & CO. INC.
126, East Lincoln Avenue P.O. Box 2000
Rahway New Jersey 07065-0900 (US)

(72) Inventor: Dixon, Richard A.F.
758 Hartley Drive
Lansdale, PA 19446 (US)

Inventor: Patchett, Arthur A.
1090 Minisink Way
Westfield, NJ 07090 (US)
Inventor: Strader, Catherine D.
119 Morningside Road
Verona, NJ 07044 (US)
Inventor: Sugg, Elizabeth E.
4108 Livingstone Place
Durham, North Carolina 27707 (US)
Inventor: Sigal, Irving S.
Deceased (US)

(74) Representative: Thompson, John Dr. et al
Merck & Co., Inc. European Patent
Department Terlings Park Eastwick Road
Harlow, Essex CM20 2QR (GB)

## (54) Modified beta adrenergic receptor.

(57) A Modified Beta Adrenergic Receptor having the amino acid residue in position 113 (aspartic acid) substituted by a serine residue has been synthesized. This modified receptor interacts with novel ligands that do not react with the natural Asp 113 containing receptor. Transgenic animals containing this modified receptor will respond to novel ligands which will not interact with the normal receptor.

EP 0 453 119 A1

## BACKGROUND OF THE INVENTION

Although hormone and drug receptors have been studied for most of this century it was the recent development of ligand-binding techniques that has permitted direct assay of the binding function of receptor macromolecules. Of the membrane bound hormone and drug receptors considerable attention has been directed to the adenylyl cyclase-coupled beta-adrenergic receptors because of their ubiquity and the diversity of the physiological responses they mediate; the close coupling of the receptors to a well-defined biochemical effector unit, the enzyme adenylyl cyclase, which has facilitated the use of the beta-adrenergic receptor (ßAR) adenylyl cyclase system as a model for studying the biochemical mechanisms of hormone receptor-effector coupling; and the therapeutic consequences of stimulation or blockade of these receptors by pharmacological means, which have implications for the therapy of a wide variety of human illnesses and to control the growth and metabolism of animals.

The ß-adrenergic receptor (ßAR) is a member of a large class of hormone receptors that are located at the plasma membrane and that exert their intracellular effects through an interaction with guanine nucleotide binding proteins (G proteins). Different G-protein-linked receptors recognize different ligands as agonists and stimulate distinct classes of G proteins. In the case of ßAR, upon binding catecholamine agonists, the receptor catalyzes the formation of the GTP complex of $G_S$, which in turn stimulates adenylyl cyclase activity. ßAR antagonists appear to act by competing with agonists for binding to the receptor and failing to stimulate adenylyl cyclase. Two subtypes of ßAR occur in mammalian tissue. The $ß_2$ subtype, found to predominate in lung tissue, binds epinephrine preferentially to norepinephrine, whereas the $ß_1$ subtype, which predominates in heart tissue, binds both agonists with similar affinities.

Dixon et al., Nature 321, 75-79, 1 May 1986, reported the cloning of the mammalian $B_2$AR gene, its cDNA sequence and its predicted amino acid sequence. Deletion mutagenesis experiments have demonstrated that the binding site of the ß-adrenergic receptor involves the hydrophobic core of the protein [Dixon et al. (1987) Nature 326, 73-77]. Single amino acid replacements for the conserved Asp 79 and Asp 113 within this putative transmembrane region had profound effects on the ability of the receptor to bind radiolabeled ligands [Strader et al. (1987) Proc. Natl. Acad. Sci. 84, 4384-4388].

The use of ßAR agonists to enhance food efficiency and carcass composition of food animals is a known technique, ß-agonists and their Effects On Animal Growth and Carcass Quality, J.P. Hanrahan, ed., Elsevier Appl. Sci. Press, New York, 1987. The presence of even residual quantities of these agonists in meat, however, is undesirable because of possible physiological effects on the natural ßAR of human consumers.

## SUMMARY OF THE INVENTION

It has now been found that substitution of the aspartate-113 residue of the ßAR receptor with serine (Ser) yields a modified ßAR that responds poorly or not at all to classical adrenergic ligands such as catecholamines but does respond to ligands that do not react with unmodified or natural ßAR. The modified ßAR can be expressed in transgenic animals using muscle specific promoters so that the animals grow in response to novel ligands. The ßARs and coupled metabolic systems of unmodified species including man, as well as the unmodified ßARs of the transgenic animal, are unaffected by the ingestion of these ligands.

## OBJECTS OF THE INVENTION

It is, accordingly, an object of the present invention to provide a novel ßAR that reacts with ligands that do not react with the natural (wild-type) ßAR. Another object is to provide vectors containing the genes that express these novel ßARs. Still another method is to provide transgenic animals containing these novel ßARs. Yet another object is to provide a method for enhancing food efficiency and carcass composition of food animals by means of ßAR agonists that do not react with the ßAR of human consumers. These and other objects of the present invention will be apparent from the following description.

## DETAILED DESCRIPTION OF THE INVENTION

The present invention is based on the finding that a change in a specific amino acid of the ßAR produces a modified receptor that will interact with ligands that do not react with the unmodified or natural ßAR. The specific amino acid of the ßAR that is changed according to the present invention is the aspartic acid residue in position 113.

This aspartic acid residue can be substituted with serine to provide a novel modified or mutant receptor that responds to ligands that do not react with unmodified or natural (wild-type) ßAR.

According to the invention described herein we have engineered the ßAR by replacing the $Asp^{113}$ residue with serine, and we have identified compounds which act as agonists for this mutant receptor while not stimulating the wild-type receptor. Thus, alterations in the design of the ligand binding pocket of the receptor, accompanied by complementary alterations in the functional groups on the ligand have resulted in the creation of new positive interactions between a receptor and a ligand

The following examples illustrate the present inventions without, however, limiting the same thereto.

EXAMPLE 1

Mutagenesis of the ßAR gene. The cloning of the cDNA for the hamster ß-adrenergic receptor (BAR) has been described by Dixon et al., Nature (1986) supra. For the introduction of the mutation into the ßAR, the cDNA was cloned into the EcoRI site of M13 mp8 and single stranded phage DNA containing the noncoding strand of the ßAR cDNA was isolated by standard procedures as described by Maniatis et al., (1982) Molecular Cloning: A Laboratory Manual (Cold Spring Harbor Laboratory, Cold Spring Harbor, N.Y.). Other standard cloning techniques were performed as described by the former Manual and would be easily reproduced by anyone trained in molecular biology. An oligonucleotide 19 nucleotides in length complementary to the phage DNA surrounding the codon for $Asp^{113}$ were synthesized using an Applied Biosystems Model 3A DNA synthesizer. The oligonucleotide contained mismatched positions in order to convert the codon for $Asp^{113}$ (GAT) to Ser (GACTTCCATTTCTGTGTTA). The oligonucleotide was annealed to the phage DNA for priming and extended using Klenow DNA polymerase and T4 DNA ligase. The double stranded DNA was digested with AccI and KpnI and the mutated fragment was ligated to the mammalian expression vector pSVHAC [Dixon et al., Nature (1987), supra) which was also digested with AccI and KpnI. pSVHAC contains the full length hamster $ß_2AR$ cDNA cloned into the EcoRI site of pSVL. pSVL contains the SV40 origin of DNA replication and a modified RNA splicing sequence [Okayama et al. (1983) Mol. Cell. Biol. 3, 280-289). The ßAR gene in this vector utilizes the SV40 early promoter, the modified splice signal, and the ßAR polyadenylation site for expression. The ligated mixture was transformed into E. coli strain DH5 (Bethesda Research Laboratories) and ampicillin resistant colonies were screened by filter hybridization using the oligonucleotide corresponding to the mutation as the hybridization probe. Plasmids were isolated from several hybridization positive colonies and mapped to insure the proper DNA insert was contained in the clone. The mutations were resolved by retransformation of the plasmid DNA into E. coli. Mutant plasmid containing colonies were again isolated by filter hybridization as above. Plasmid DNA was isolated from overnight cultures of cells by alkaline lysis and twice banding in CsCl-ethidium bromide equilibrium density gradients. The region of each mutant plasmid which was subcloned into the vector was sequenced to confirm the identity of the mutation. The purity of the plasmid preparation was tested by retransformation of the DNA into E. coli followed by colony hybridization. Of the 100 colonies examined for each plasmid, all were positive by hybridization. A neomycin drug resistance marker was added to the pSVHAC plasmid containing the position 113 mutation. The Klenow DNA polymerase, blunt-ended EcoRI fragment containing the tk-neo cassette from plasmid pCMVIE-AKI-DHFR (Silberklang et al., 1987, in "Modern Approaches to Animal Cell Technology" eds. R. E. Spier and J. B. Griffiths, Butterworth and Co., U.K., pp. 199-214) was subcloned into the blunt-ended HindIII site of the mutant-containing plasmids. Plasmid DNA was isolated as above and confirmed by restriction mapping.

EXAMPLE 2

Expression in mammalian cells. For the establishment of clonal cell lines expressing mutant receptors, the plasmid DNAs were transfected into mouse L cells by the $CaPO_4$ procedure. The DNA (15mg) in HEPES buffered saline was precipitated with 125 mM $CaCl_2$ at room temperature for 20 min. The precipitate was added to the media covering subconfluent monolayers of mouse L cells in 100 mm petri dishes and incubated at 37°C for 4 hrs. The media was removed and the cells were washed once with Dulbecco's modified minimal essential medium (DMEM). DMEM containing 15% glycerol added to the monolayer for 1 min followed by three washes with DMEM. DMEM containing 10% fetal bovine serum was then added to the monolayers and the cells were incubated at 37°C for 12-18 hrs. The medium was changed to DMEM containing 10% fetal bovine serum and 500 mg/ml genticin to select for cells containing the expression vector. The cells were then incubated at 37°C for 1-2 weeks to allow genticin-resistant colonies to form. Individual colonies were isolated and expanded into cell lines.

L cells expressing the wild-type receptor were identified by $^{125}I$-cyanopindolol ($^{125}I$-CYP) binding, as described below. L cells expressing the mutant receptors did not bind $^{125}I$-CYP at detectable levels and were identified by indirect immunofluorescence using a monoclonal antibody which recognizes the ßAR. The antibody was raised against a peptide corresponding to a region of the proposed 3rd intracellular loop of the hamster

ßAR, with the sequence Y-A-K-R-Q-L-Q-K-I-D-K-S-E-G-R, Zemcik et al. (1988) Biochem. J. 251, 333-339. Cell monolayers were washed in phosphate buffered saline (PBS), fixed in 3.7% formalin, 1% ethyldimethyldiaminopropyl carbodiimide in PBS for 10 min at 23°C. After permeabilization in 0.1% Triton X-100 for 5 min, cells were treated with 4 mg/ml of goat gamma globulin for 1 hr at 23°C to reduce nonspecific interactions, then washed and incubated with the monoclonal antibody at 0.1 mg/ml for 1 hr at 23°C. After further washing in PBS, cells were incubated with FITC-conjugated goat anti-mouse IgG at a 1:50 dilution for 1 hr at 23°C. Cells were then washed extensively in PBS and the fluorescence observed using a Leitz Diaplan fluorescence microscope.

EXAMPLE 3

Membrane Preparation. COS-7 cell monolayers were washed 3 times in PBS, scraped from the flask with a rubber policeman, and centrifuged at 2000 x g for 5 min. The cell pellet was resuspended in 75 mM Tris, pH 7.4, 12.5 mM $MgCl_2$, 1.5 nM EDTA (TME buffer) at a concentration of $10^8$ cells/ml, then diluted 1:5 with water and lysed by freezing in liquid $N_2$ and thawing with vigorous vortexing. Nuclei were removed by centrifugation over a cushion of 60% sucrose at 2500 x g for 15 min. The upper layer was collected, membranes pelleted by centrifugation at 40,000 x g for 15 min,and resuspended in TME at a protein concentration of 1-2 mg/ml.

Membranes were prepared from L cell monolayers by hypotonic lysis in 10 mM Tris, pH 7.5 for 10 min at 4°C. Cells were then scraped from the flask in the hypotonic buffer, membranes pelleted by centrifugation at 40,000 x g for 15 min, and resuspended at a protein concentration of 0.5-2 mg/ml in TME buffer.

EXAMPLE 4

Receptor Assays. $^{125}$I-CYP binding to membranes was measured in a final volume of 250 ul of TME buffer, containing 10-20 mg of membrane protein and 225 pM $^{125}$I-CYP for 90 min at 23°C, as previously described Dixon et al. (1987), supra. Membranes were collected by filtration on GF/C glass fiber filters and bound $^{125}$I-CYP was detected with a gamma counter. Nonspecific binding, measured in the presence of 10 mM alprenolol, was subtracted from the total binding.

Adenylyl cyclase activity was determined by the method of Saloman et al., (1974) Anal. Biochem. 58, 541-548. Briefly, 20 ml of TME buffer containing 5-20 mg of membrane protein was mixed with 30 ml of a solution containing 7 mM phosphoenolpyruvate, 0.3 mM ATP (with $10^6$ cpm of a-$^{32}$P-ATP), 0.3 mM cAMP, 0.1 mM GTP, 1.5 units of mykokinase, and 0.3 units of pyruvate kinase. The reaction was allowed to proceed for 30 min at 30°C before it was quenched at 4°C with an excess of unlabeled ATP. The $^{32}$P-cAMP produced was determined by Dowex and alumina chromatography, as described by Salomon et al., supra. For stimulation of the enzyme, test compounds were included in the assay at the concentrations from $10^{-10}$ to $10^{-3}$ M. Maximal stimulation was achieved with 10 mM NaF.

EXAMPLE 5

Data Analysis. Activation constant ($K_{act}$) values for adenylyl cyclase stimulation were obtained by computer assisted nonlinear regression analysis of curves generated in the presence of increasing concentrations of compounds. The Michaelis-Menton equation was used: $V=V_{max}[A]/K_{act}+[A]$, where V is the level of cyclase stimulation (in pmol $^{32}$P-cAMP/mg protein/min) measured at a given agonist concentration [A], and $V_{max}$ is the calculated maximal stimulation at infinite agonist concentration. In cases where the stimulation curve did not reach a plateau at experimentally assessible concentrations of a compound, Vmax was assumed to be equal to the maximal stimulation achieved with 10 mM NaF, as explained in the legend to Table 1.

EXAMPLE 6

Potein Immunoblotting. COS-7 membranes containing 1 mg of membrane protein were pelleted by centrifugation at 15,000 x g for 30 min and resuspended at a concentration of 10-20 mg/ml in 65 mM Tris, pH6.3, 3% SDS, 10% glycerol, 5% 2-mercapto-ethanol. Proteins were denatured at 4°C for 1 hr and separated on 10% polyacrylamide gels by the method of Laemmli (1970) Nature 117, 680-685. After electrophoresis, proteins were transferred to nitrocellulose and the immunoreactive receptor detected by immunoblotting Towbin et al., (1979) Proc. Natl. Acad. Sci. (USA) 76, 2974-2978. The antibody used in immunoblotting was raised to a peptide corresponding to the C-terminus of the hamster ßAR, with the sequence C-L-D-S-Q-G-R-N-Nle-S-T-N-D-S-P-L, used at a serum dilution of 1:1000, followed by $^{125}$I-Protein A at a final concentration of $10^6$ cpm/ml.

RESULTS

The expression of mutant ßARs having Ser at position 113 was first assessed in membranes from COS-7 cells by protein immunoblotting. Transfection of these cells with the wild-type (natural) receptor resulted in the production of an immunoreactive protein which migrates with an apparent molecular weight of 68 kDa. The appearance of this fully glycosylated form of the receptor was previously determined to indicate proper folding and processing of the protein in the cell membrane (Strader et al., supra). [$Ser^{113}$]ßAR comigrated with the wild-type ßAR, suggesting that this amino acid substitution did not affect the folding of the protein in the cell membrane. However, $^{125}$I-CYP binding could be measured only for the wild-type receptor, as [$Ser^{113}$]ßAR did not bind detectable levels of the antagonist.

The interactions of adrenergic ligands with mutant receptors which bind ligands with reduced affinity can be detected by measuring the ability of these compounds to act as agonists to mediate adenylyl cyclase stimulation by the mutant receptors. Thus, it has been found that [$Glu^{113}$]ßAR and [$Asn^{113}$]ßAR, which do not bind $^{125}$I-CYP with an affinity high enough to be detected by equilibrium binding measurements, are able to interact at low affinity with ß-adrenergic agonists to stimulate adenylyl cyclase activity (Strader et al., J. Biol. Chem. 263:10267-10271 (1988). The interactions of mutant receptors with Ser at position 113, expressed in mouse L-cells, with several different compounds were examined by this method, with the results summarized in Table 1. The agonist isoproterenol binds to and activates the wild-type receptor with a $K_{act}$ of 2 x $10^{-8}$ M. The affinity of isoproterenol for the receptor is decreased 5000-fold by the substitution of a Ser residue for $Asp^{113}$ (Table 1).

TABLE 1

| Compound | Wild-type ßAR %NaF | Wild-type ßAR Kact(M) | [Ser$^{113}$]ßAR %NaF | [Ser$^{113}$]ßAR Kact(M) |
|---|---|---|---|---|
| 1) 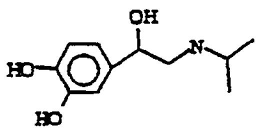 isoproterenol | 100 | $2x10^{-8}$ | 37 | $10^{-3}$ |
| 2) 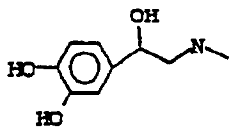 epinephrine | 100 | $3x10^{-7}$ | 30 | $10^{-3}$ |
| 3) 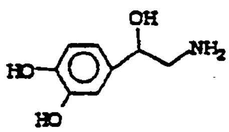 norepinephrine | 100 | $3x10^{-6}$ | 18 | $10^{-3}$ |
| 4) 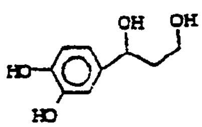 | 10 | nd | 10 | nd |
| 5) 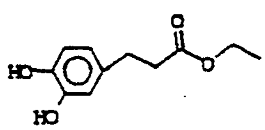 L-684, 282 | 3 | nd | 62 | $1.2x10^{-4}$ |
| 6) 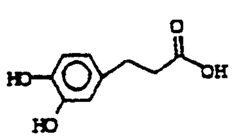 L-591, 530 | nd | nd | 25 | $10^{-3}$ |

| | | | | |
|---|---|---|---|---|
| 7) 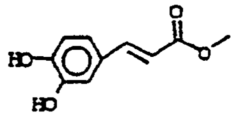 L-714,010 | 0 | - | 40 | $3x10^{-4}$ |
| 8) 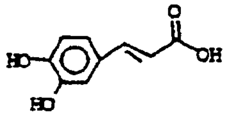 L-583,991 | nd | nd | 0 | - |
| 9) 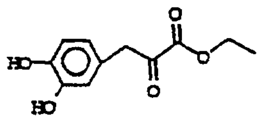 L-349,563 | nd | nd | 25% | $3x10^{-4}$ |
| 10) 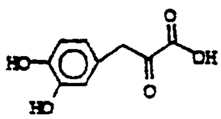 L-586,462 | 0 | - | 0 | - |
| 11) 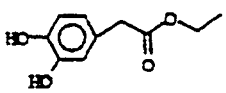 L-158,799 | 20 | $10^{-3}$ | 44 | $1.9x10^{-4}$ |
| 12) 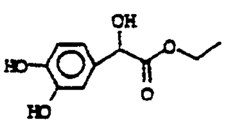 L-158,755 | 0 | nd | 12 | nd |
| 13) 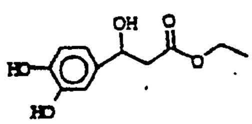 | 7 | nd | 36 | $1.8x10^{-4}$ |
| 14) 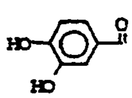 | nd | nd | 5 | nd |
| 15) 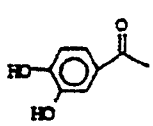 L-341,829 | 0 | nd | 28 | $10^{-3}$ |

| | | | | |
|---|---|---|---|---|
| 16) 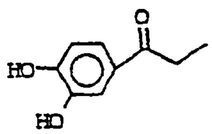<br>L-584,553 | nd | nd | 50 | $5x10^{-4}$ |
| 17) 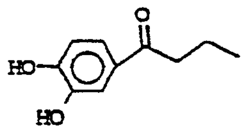<br>L-574,529 | 5 | nd | 90 | $2x10^{-4}$ |
| 18) 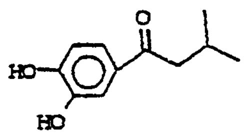<br>L-158,870 | 13 | $10^{-3}$ | 106 | $4x10^{-3}$ |
| 19) 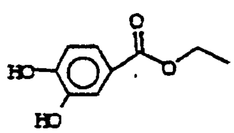 | 0 | nd | 75 | $1.1x10^{-4}$ |

This mutant receptor maintains its ß$_2$AR-like affinity for the endogenous agonists epinephrine (Table 1, compound 2) and norepinephrine (Compound 3), however, in that epinephrine is more potent than norepinephrine in activating the receptor, although the affinity for both ligands is decreased 5000-fold relative to the wild-type protein. In contrast to the wild-type ßAR, [Ser$^{113}$]ßAR is activated by catechol esters, such as compounds 5, 7, 9, 11, 13, and (weakly) 12 in Table 2. Acids are less effective than esters in causing this activity, as demonstrated by a comparison of compound 6 (acid) with 5 (ester), of compound 8 (acid) with 7 (ester), and of compound 10 (acid) with 9 (ester). This may result from a decreased recognition of acids by other amino acids in or near binding site of the mutant receptor, since the wild-type receptor normally binds positively-charged amines. Esters having a 2-carbon chain between the catechol ring and the ester moiety are more active than those with only a 1-carbon chain (compare compounds 5 and 11, and compounds 13 and 12). Substitution of a ketone or an ester on the -carbon of the chain enhances activity (compounds 14-19), and increasing the hydrophobicity of the alkyl chain results in increased activity. These results suggest that the placement of the ester or ketone in the binding site of the receptor is critical for activity. The observation that these compounds activate [Ser$^{113}$]ßAR and not the wild-type receptor demonstrates that an interaction between the ester and the serine residue at position 113 can activate this mutant receptor in a manner analogous to the activation of the wild-type receptor by an interaction between the protonated amine of catecholamine agonists and the aspartic acid residue at position 113.

## EXAMPLE 7

The muscle-specific expression of luciferase in transgenic mice has been reported by DiLella et al., Nucleic Acids Research 16:4159 (1988). Using similar techniques an expression plasmid is prepared for the modified ßAR receptor containing mutations at the codon for amino acid 113 as previously described. This plasmid contains a skeletal muscle specific promoter, muscle actin, so that, due to the tissue specific expression of the promoter, the ßAR is expressed only in skeletal muscles (Table 2). The plasmid is prepared by substituting the muscle actin promoter for the promoter in pSVL. The plasmid is injected into embryos of the selected avian or mammalian species, e.g., chickens, ducks and turkeys, or pigs, cattle and sheep, which are then transplanted into the mother or a surrogate. Progeny are screened for presence of the input plasmid DNA using oligonucleotide hybridization for the specific mutant ßAR gene (Dixon et al., Nature, 1987, supra). DNA positive individuals are assayed by immunoblotting for expression of the ßAR protein and by $^{125}$I-cyanopindolol binding and adenylyl cyclase activation by isoproterenol or by compound #18 (Table 1) (Strader et al., Proc. Natl. Acad.

Sci. USA, supra). Animals demonstrating expression of the ßAR protein are cross-bred to produce stable populations of expressing animals. Animal lines expressing the mutated ßAR are established and tissues from the animals are assayed for adenylyl cyclase responses to compounds specific for ßAR and each of the mutants. Where positive responses are obtained, the animals are treated with ligands specific for that mutant ßAR and in vivo responses measured.

Expression of the wild-type ßAR in the muscle tissue of transgenic mice, driven by the skeletal actin promoter, is demonstrated in Table 2. Expression levels in the muscles of the transgenic mice are 7x higher than in controls, with no difference in the receptor levels in other tissues.

Table 2

Expression of the Wild-type ßAR in Transgenic Mice

| mouse | $^{125}$I-CYP binding (fmol/mg) | | | |
|---|---|---|---|---|
| | skeletal muscle | lung | liver | heart |
| control (n=8) | 16.5±5 | 124±50 | 2.1±2.6 | 10.5±3 |
| transgenic (n=5) | 119±35 | 115±96 | 2.3±3.4 | 12.2±5 |

Legend: Tissues were removed from mice, frozen in liquid $N_2$, then thawed, homogenized in TME buffer (Example 3), and membranes isolated by centrifugation on a 50% sucrose cushion. $^{125}$I-CYP binding was performed as in Example 4.

## Claims

1. A method of producing transgenic animals whose growth and metabolic characteristics can be favorably controlled, which method comprises introducing into the genetic line of such animals a mutant ß-adrenergic receptor whose activation requires the administration of a ligand specifically able to stimulate this receptor without affecting normal ß-adrenergic receptors in the transgenic animal or other species.

2. A vector comprising a ß-adrenergic receptor modified by replacement of the aspartic acid residue at position 113 by serine, operatively linked to a muscle specific promoter.

3. A vector according to Claim 2 wherein the promoter is muscle actin.

4. An embryo of a member of an avian or mammalian human food species that has been transformed by incorporation of the vector of Claim 2 or Claim 3.

5. An avian embryo according to claim 4 wherein the species is chicken, duck or turkey.

6. A mammalian embryo according to claim 4 wherein the species is cattle, pig or sheep.

7. A method of promoting growth in a transgenic member of an avian or a mammalian human food species that has been transformed with a vector of Claim 1 comprising administering to the member an agonist that does not react with a normal ß-adrenergic receptor but which reacts with the modified receptor expressed by the transgenic member.

8. A method according to claim 7 wherein the agonist is

1) 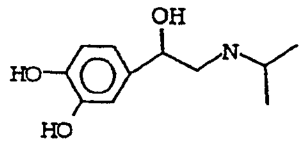

2) 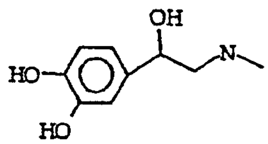

3) 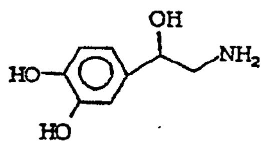

4) 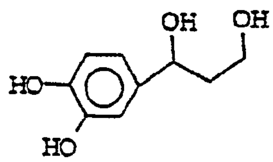

5) 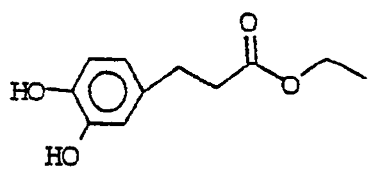

6) 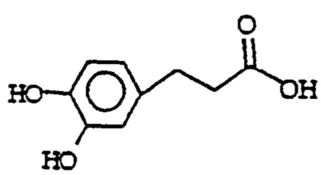

7) 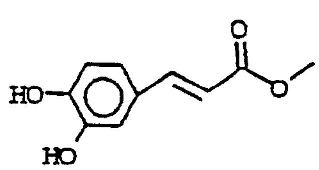

8) 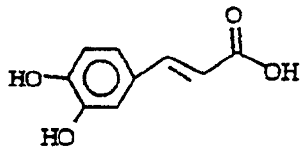

9) 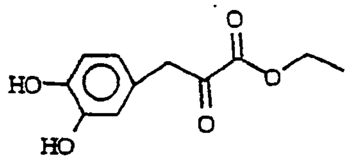

10) 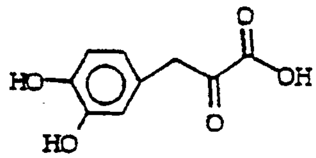

11) 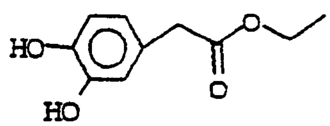

12) 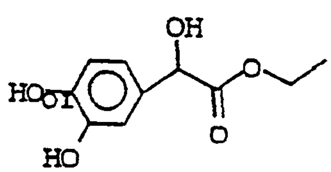

13) 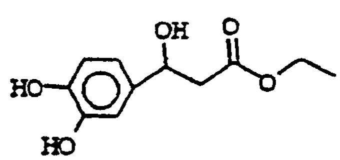

14) 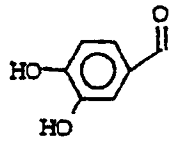

15) 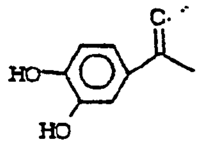

16) 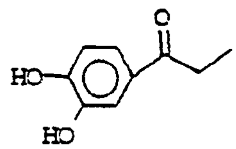

17) 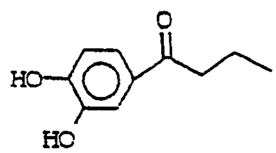

18) 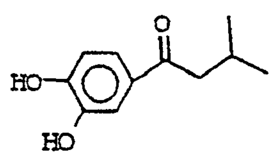

19) 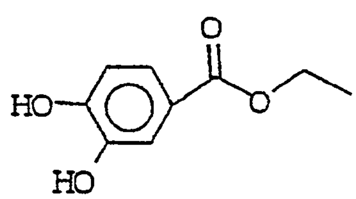

**9.** A member of an avian or a mammalian human food transgenic species having in its genome a ß-adrenergic receptor modified by replacement of the aspartic acid residue at position 113 by serine.

**10.** A ß-adrenergic receptor modified by replacement of the aspartic acid residue at position 113 by serine.

European Patent Office

# EUROPEAN SEARCH REPORT

Application Number

EP 91 30 2843

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 351 921 (MERCK & CO.)(24-01-1990)<br>* Whole document * | 1-10 | C 07 K 13/00<br>C 12 N 15/12<br>C 12 N 15/85<br>A 01 K 67/00<br>A 61 K 31/05<br>C 12 N 15/00 |
| D,X | THE JOURNAL OF BIOLOGICAL CHEMISTRY, vol. 263, no. 21, 25th July 1988, pages 10267-10271; C.D. STRADER et al.: "Conserved aspartic residue 79 and 113 of the beta-adrenergic receptor have different roles in receptor function"<br>* Abstract; discussion * | 10 | |
| D,Y | IDEM | 1 | |
| Y | TRENDS IN BIOTECHNOLOGY, no. 1, January 1987, pages 13-19, Amsterdam, NL; R.B. CHURCH: "Embryo manipulation and gene transfer in domestic animals"<br>* The whole document * | 1 | |
| A | POULTRY SCIENCES, vol. 65, no. 8, 8th August 1986, pages 1437-1444; R.M. SHUMAN et al.: "Gene transfer by avian retroviruses"<br>* The whole document * | | TECHNICAL FIELDS SEARCHED (Int. Cl.5)<br>C 07 K<br>C 12 N |
| P,X | J. BIOL. CHEM., vol. 266, no. 1, 1991, pages 5-8; M. CANDELORE et al.: "Allele-specific activiation of genetically engineered receptors"<br>* The whole document * | 1-10 | |

The present search report has been drawn up for all claims

| Place of search | Date of completion of the search | Examiner |
|---|---|---|
| THE HAGUE | 11-07-1991 | NAUCHE S.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)